# EUROPEAN PATENT APPLICATION

(11) **EP 0 930 314 A1**
(43) Date of publication of application: **21.07.1999**
(21) Application number: 97933907.4
(22) Date of filing: 05.08.1997
(51) Int. Cl.: C07D 491/113, A61K 31/445

(54) **p-TOLUENESULFONATE HYDRATE OF THIAZOLINE COMPOUND**

(30) Priority: 06.08.1996 JP 20629096
(71) Applicant: TAISHO PHARMACEUTICAL CO. LTD, Tokyo 171 (JP)
(72) Inventor: HATAYAMA, Katsuo, Deceased (JP); SATO, Masakazu, Taisho Pharmaceutical Co., Ltd., Tokyo 171 (JP); MANAKA, Akira, Taisho Pharmaceutical Co., Ltd., Tokyo 171 (JP); TAKAHASHI, Keiko, Taisho Pharmaceutical Co., Ltd., Tokyo 171 (JP); KAWASHIMA, Yutaka, Taisho Pharmaceutical Co., Ltd., Tokyo 171 (JP); HATAYAMA, Sachiko, Hasuda-shi, Saitama 330 (JP)
(74) Representative: Weisert, Annekäte, Dipl.-Ing. Dr.-Ing.
(86) International application number: JP9702715
(87) International publication number: WO9805668

(57) **Abstract**

p-Toluenesulfonate dihydrate of a thiazoline compound represented by the formula: and a fibrinogen receptor antagonist comprising the same as an effective component.

The present invention provides a compound which has an excellent fibrinogen receptor antagonism, and is easy to store and to produce the pharmaceutical preparation for oral administration because of the compound's non-hygroscopic property.

## Description

### TECHNICAL FIELD

The present invention relates to a salt hydrate of a thiazoline compound having an inhibitory action of blood platelet aggregation, etc. because of having an fibrinogen receptor antagonism.

### BACKGROUND ART

Blood platelet aggregation is considered to result from the binding among blood platelets via fibrinogen at the binding site to fibrinogen on the blood platelet membrane glycoprotein GPIIb/IIIa complex which originates by stimulation of various blood platelet aggregation-inducing substances.

A compound represented by the following Formula (1) disclosed in Japanese Patent Application Kokai No. 7-242646 is remarkably useful for various diseases associated with fibrinogen receptors because of having an excellent fibrinogen receptor antagonism.

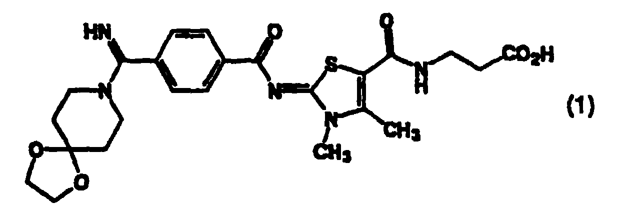

However, the compound of Formula (1) is hygroscopic, therefore it is insufficient for storing or producing the pharmaceutical preparation for oral administration.

An object of the present invention is to provide a compound which has an excellent fibrinogen receptor antagonism, and is easy to store and to produce the pharmaceutical preparation for oral administration because of the compound's non-hygroscopic property.

### DISCLOSURE OF THE INVENTION

As a result of extensive researches on various salts and hydrates of the compound of Formula (1) for solving the above-mentioned problem, the present inventors have found that p-toluenesulfonate dihydrate of the compound of Formula (1) not specifically described in Japanese Patent Application Kokai No. 7-242646, particularly dihydrate thereof, has an excellent fibrinogen receptor antagonism and non-hygroscopic property, and thus the present invention has been accomplished.

Accordingly, the present invention is directed to p-toluenesulfonate dihydrate of the compound of Formula (1) and is directed to a fibrinogen receptor antagonist containing the same as an effective component.

The p-toluenesulfonate dihydrate of the present invention can be prepared by the same method as an ordinary method for preparing a salt from the compound of Formula (1) or a salt (e.g. sodium salt) thereof, preferably by the following method for industrial use.

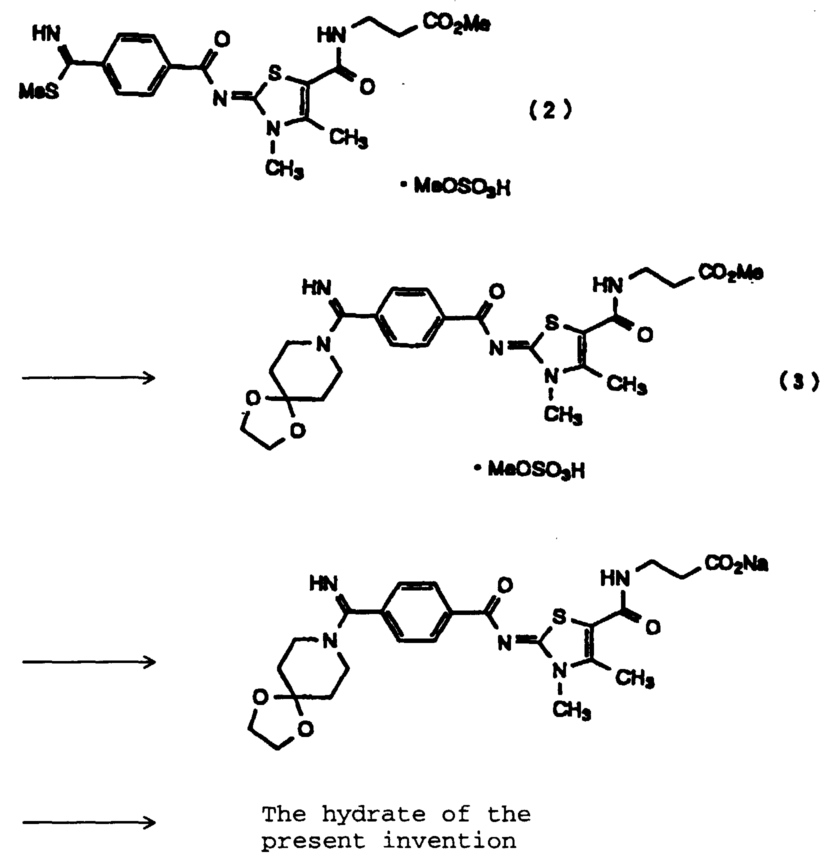

N-(2-Methoxycarbonylethyl)-2-[4-(methylthioimidoyl)benzoylimino]-3,4-dimethyl-3H-thiazoline-5-carboxamide methylsulfate (2) described in Reference Example 1 of WO95/19360 can be used as a starting material. The compound of Formula (2) is reacted with 1,4-dioxa-8-azaspiro[4,5]decane to give a compound of Formula (3), which is then hydrolyzed in the presence of sodium hydroxide, thereby there is obtained sodium salt of the compound of Formula (1) which is preferable for forming p-toluenesulfonate. Sodium salt of the compound of Formula (1) is reacted with an agueous solution of p-toluenesulfonic acid at room temperature to give a hydrate of the present invention. The hydrate obtained herein has an industrially sufficient purity by even washing with water.

### BRIEF DESCRIPTION OF THE DRAWING

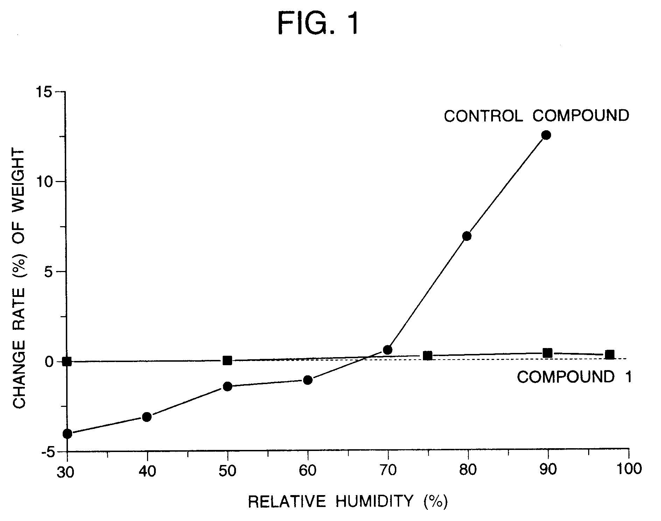

Fig 1 is a graph showing a result of hygroscopicity test of samples obtained in the experiment wherein the change rate of weight and the relative humidity are plotted on the ordinate and abscissa, respectively.

### BEST MODE FOR CARRYING OUT THE INVENTION

The hydrate of present invention is illustrated in more detail by the following example and experiments.

### Example

(1) To a heated (50°C) mixture of 1,4-dioxa-8-azaspiro[4,5]decane (52.1 g), acetic acid (20.8 ml) and acetone (1100 ml) was added N-(2-methoxycarbonylethyl)-2-[4-(methylthioimidoyl)benzoylimino]-3,4-dimethyl-3H-thiazoline-5-carboxamide methylsulfate (153 g) with stirring, followed by stirring under heating reflux for 2 hours. The reaction mixture was cooled on ice, and the precipitated crystals were collected by filtration and washed with acetone (400 ml). The resulting crude crystals were dissolved in hot methanol (1500 ml), the insoluble matters were removed by filtration, and the filtrate was concentrated to about one fourth volume under reduced pressure. Toluene (1000 ml) was added to the resulting suspension, the mixture was stirred at 80°C for an hour and under ice-cooling for an hour, and the precipitated crystals were collected by filtration to give N-(2-methoxycarbonylethyl)-2-{4-[(4,4-ethylenedioxypiperidin-1-yl)imidoyl]benzoylimino}-3,4-dimethyl-3H-thiazoline-5-carboxamide methylsulfate (149.8 g).
   m.p. 215 ― 216°C (decomposed).
(2) A mixture of the compound (102 g) obtained above, sodium hydroxide (14 g), water (280 ml) and 2-propanol (1000 ml) was stirred at room temperature for 110 minutes. The precipitated crystals were collected by filtration and washed with 2-propanol to give sodium 3-{2-{4-[(4,4-ethylenedioxy)piperidin-1-yl]imidoylbenzoylimino}-3,4-dimethyl-3H-thiazoline-5-carbonylamino}-propionate (65.5 g).
   m.p. >300°C
   ¹H-NMR (DMSO-d₆) δ (ppm):
      1.62 (4H, m), 2.09 (2H, t, J=6 Hz), 2.62 (3H, s), 3.31 (4H, q, J=6 Hz), 3.33 (4H, m), 3.82 (3H, s), 3.89 (4H, m), 7.47 (2H, d, J=8 Hz), 8.25 (2H, d, J=8 Hz), 9.00 (1H, t, J=6 Hz)
(3) The compound (30 g) obtained in the above (2) was added to an aqueous solution (600 ml) of p-toluenesulfonic acid monohydrate (23.4 g), followed by stirring at 19 ― 20°C for 2 hours. The precipitated crystals were collected by filtration, washed with water and dried to give N-(2-carboxyethyl)-2-{4-[(4,4-ethylenedioxy)piperidin-1-yl]imidoylbenzoylimino}-3,4-dimethyl-3H-thiazoline-5-carboxamide p-toluenesulfonate dihydrate (37.2 g, hereinafter referred to as "Compound 1").
   m.p. 133 ― 133.5°C.

### Experiment 1

Compound 1 obtained in the above example and dihydrate of the compound of Formula (1) were used as test drugs. About 100 mg of the test drug was taken into a weighing bottle and weighed accurately. The weighed test drug in the weighing bottle was stored at 25°C in a decicator which was adjusted to the defined relative humidity by using a desired saturated salt solution or a thermo-hygrostat (Tabaiestec SH-220) which was adjusted to the proper relative humidity.

After storage for 30 days, the test drug was weighed, and the change rate of weight was calculated to use as an index of hygroscopicity. Results were shown in Fig 1.

### Experiment 2 [Fibrinogen Receptor (GPIIb/IIIa) Binding Inhibition Test]

Purified GPIIb/IIIa was prepared by referring to the methods of Pytela et al (Science, 231 (1986) 1559) and a binding measurement system of solid phased GPIIb/IIIa was carried out by referring to the method of Kouns et al (Blood, 80 (1992) 2539).

Purified GPIIb/IIIa receptor was solidified on 96-well microtiter plates by reacting in a solution (pH 7.4) containing 20 mM Tris-hydrochloric acid, 150 mM sodium chloride, 1 mM calcium chloride, 1 mM magnesium chloride and 0.0005 % Triton X-100 at 4°C for 16 hours. The solid phase was then washed once with a solution (pH 7.4) containing 20 mM Tris-hydrochloric acid, 150 mM sodium chloride, 1 mM calcium chloride, 1 mM magnesium chloride and 0.05 % Tween 20 (Buffer B), and blocked by a solution (pH 7.4) containing 20 mM Tris-hydrochloric acid, 150 mM sodium chloride, 1 mM calcium chloride, 1 mM magnesium chloride and 25 % Block Ace in an amount of 150 µL/well at room temperature for 2 hours. After washing with Buffer B once, the resulting matter was reacted with 100 µL/well of a mixture of 2 µg/ml of fibrinogen in a solution (pH 7.4) containing 20 mM Tris-hydrochloric acid, 150 mM sodium chloride, 1 mM calcium chloride, 1 mM magnesium chloride and 10 % Block Ace and an inhibitor at a defined concentration at 4°C for 16 hours, and washed with Buffer B three times. The amount of the fibrinogen bound to the plates was measured by an enzyme-linked immunosorbent assay method using antifibrinogen primary antibody and peroxidase-labelled secondary antibody to calculate IC₅₀ value.

The measurement results of Compound 1 and GRGDS (Gly-Arg-Gly-Asp-Ser; produced by Sigma Co.) used as a reference drug are shown in Table 1.

**Table 1**

| Test drug | IC₅₀ value (M) |
|---|---|
| Compound 1 | 8.05 × 10⁻¹¹ |
| GRGDS | 6.80 × 10⁻⁷ |

### Experiment 3 [Human in vitro Blood Platelet Aggregation Inhibition Test]

Citrated blood (the volume ratio of 3.13 % sodium citrate solution to blood is 1:9) was collected from the cubital vein of a healthy human who had not been received any drugs known to affect the function of blood platelet within 2 weeks prior to starting the test. The blood was centrifuged at 120 x g at room temperature for 15 minutes to give platelet rich plasma (PRP) as a supernatant, and further centrifuged at 1500 x g for 10 minutes to give platelet poor plasma (PPP) as a supernatant.

The blood platelet counts of PRP were adjusted to 50 ― 60 x 10⁴/µL by diluting with PPP.

Blood platelet aggregation was monitored according to the method of Born G.V.R., [Nature, vol. 194, page 927 (1962)] using adenosine diphosphate (produced by Sigma Co.; hereinafter referred to as "ADP") as an aggregation-inducing substance. That is, a solution of Compound 1 or dihydrate of the compound of Formula (1) as a test drug in dimethyl sulfoxide was adjusted to the desired concentration with a physiological saline solution. 25 µL of the solution was added to 250 µL of PRP and incubated at 37°C for 3 minutes, and 25 µL of ADP (final concentration : 7 µM) was added thereto. The mixture was monitored for 5 minutes by using a blood platelet aggregation ability measurement apparatus (Aggricoda TM · PA-3210; made by Kyoto Daiichi Kagaku Co.) and the concentration of the test drug to inhibit the maximum aggregation by 50 % was calculated.

Results are shown in Table 2.

**Table 2**

| Test drug | IC₅₀ value (nM) |
|---|---|
| Compound 1 | 22.1 |
| Dihydrate of the compound of Formula (1) | 21.1 |

### INDUSTRIAL APPLICABILITY

The thus-obtained hydrate of the present invention inhibits a fibrinogen receptor on blood platelet (GpII/IIIa) from binding to fibrinogen, and has an excellent inhibitory action of blood platelet aggregation. Accordingly, the hydrate of the present invention can be used as a preventive and therapeutic agent for ischemic diseases (e.g. thrombosis, cerebral infarction or myocardial infarction) and arteriosclerosis diseases, and metastasis inhibitory agents of malignant tumors.

For the purposes, the hydrate of the present invention can be mixed with, for example, conventional fillers, binders, disintegrators, pH modulators or solubilizers, and prepared in the form of tablets, piles, capsules, granules, powders, solutions, emulsions, suspensions or injections by conventional formulation techniques.

The dose of the hydrate of the present invention for adult patients is 1 to 1000 mg in case of oral administration, and 0.01 to 100 mg in case of parenteral administration, in a single or several divided doses per day. This dose can be increased or decreased depending on the kind of the diseases and the age, body weight and condition of the patient.

## Claims

1. p-Toluenesulfonate dihydrate of a thiazoline compound represented by the formula:

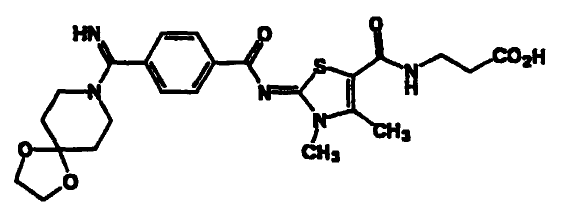

2. A pharmaceutical preparation comprising p-toluenesulfonate dihydrate of the thiazoline compound according to Claim 1.

3. Use of p-toluenesulfonate dihydrate of the thiazoline compound according to Claim 1 for a pharmaceutical preparation.

4. Use of p-toluenesulfonate dihydrate of the thiazoline compound according to Claim 1 for a fibrinogen receptor antagonist.

5. Use of p-toluenesulfonate dihydrate of the thiazoline compound according to Claim 1 for a blood platelet aggregation inhibitor.

6. The pharmaceutical preparation according to Claim 2 for a fibrinogen receptor antagonist.

7. The pharmaceutical preparation according to Claim 2 for a blood platelet aggregation inhibitor.
